# EUROPÄISCHE PATENTANMELDUNG

(11) **EP 1 188 517 A1**
(43) Veröffentlichungstag der Anmeldung: **20.03.2002**
(21) Anmeldenummer: 00119988.4
(22) Anmeldetag: 14.09.2000
(51) Int. Cl.: B24B 27/027, B24B 45/00, B24B 19/14, B24B 49/12, B23B 31/107, B23Q 17/24

(54) **Austauschbarer Werkzeugeinsatz und endoskopisches Bearbeitungsgerät**

(71) Anmelder: Storz-Endoskop GmbH, 8200 Schaffhausen (CH)
(72) Erfinder: Ruegg, Thomas, 8200 Schaffhausen (CH); Krattiger, Beat, 8222 Beringen (CH); Haan, Harald, 8200 Schaffhausen (CH)
(74) Vertreter: Heuckeroth, Volker, Dr.

(57) **Zusammenfassung**

Ein austauschbarer Werkzeugeinsatz für ein endoskopisches Bearbeitungsgerät, weist eine Welle auf, die an ihrem distalen Ende mit einer Werkzeugeinheit und an ihrem proximalen Ende mit einem Kupplungselement verbunden ist. Die Welle ist flexibel ausgebildet, und die Werkzeugeinheit weist ein Verriegelungselement auf, das mit einem Verriegelungsteil des Bearbeitungsgeräts zusammenwirkt, um den Werkzeugeinsatz lösbar zu halten.

## Beschreibung

Die Erfindung betrifft einen austauschbaren Werkzeugeinsatz (Austauschwerkzeigeinsatz) für ein endoskopisches Bearbeitungsgerät, mit einer Welle, die an ihrem distalen Ende mit einer Werkzeugeinheit und an ihrem proximalen Ende mit einem Kupplungselement verbunden ist. Ferner betrifft die Erfindung ein endoskopisches Bearbeitungsgerät mit einem vorgenannten Werkzeugeinsatz.

**Stand der Technik**
- Bei bekannten starren Geräten erfolgt die Bewegungsübertragung vom proximal gelegenen Motor zum distalen Arbeitswerkzeug mittels starren Antriebswellen oder mit Transmissionsriemen. Diese Übertragungselemente erfordern Getriebeeinheiten oder Umlenkrollen, welche Kosten Platz- und Effizienzverlust bewirken. Die Konstruktion der Energietransmission vom proximal gelegenen Motor zum distalen Arbeitswerkzeug ist kompliziert und störungsanfällig. Starre Antriebswellen, die parallel und nahe der Längsachse des Arbeitsschaftes liegen, können nicht direkt an einen Motor angekuppelt werden, da der Motor gewisse Mindestabmessungen aufweist, die mit der starren Linsenoptik kollidieren. Bei den optisch schlechter auflösenden Faseroptiken wäre dies allerdings nicht der Fall.
- Fliehkräfte und Unwuchten führen zu Vibrationen, die die Drehzahl nach oben begrenzen. Durch diese konstruktionsbedingte Niedertourigkeit folgt eine geringere Abtragsleistung.
- Das Übertragungssystem für die Bewegung des Schleifkopfes ist Verschleiss unterworfen und kann nicht bei dem Gerät "Blending Scope" der Fa. R. Wolf durch den Anwender ausgetauscht werden.
- Ein medizinische Gerät der Anmelderin muss zur Schmierung der Lager mit Spülflüssigkeit betrieben werden. Es ist daher nicht einsetzbar für technische Anwendungen. Der Austauschwerkzeugeinsatz enthält alle Verschleisselemente wie Lager und Fräskopf, sowie die Welle und den Wellenschutz. Er ist aber starr und konstruktionsbedingt nicht für hohe Tourenzahlen ausgelegt.

**Aufgabe der Erfindung**
- Endoskopische universelle Bearbeitung und /oder Analyse unter optischer Sichtkontrolle hauptsächlich mit einem rotierenden Werkzeug an schwerzugänglichen Stellen. Die Bearbeitung beinhaltet beispielsweise das Entgraten von Gussteilen, das Ausschleifen von Einschlagskerben an Triebwerksschaufeln, das Abschleifen von Korrosion zur Oberflächenanalyse z.B. bei Rissprüfungen, das Abschleifen von Schweissnähten, das Bohren von Löchern etc.
- Möglichst grosser Materialabtrag pro Zeiteinheit.
- Kostengünstiger Betrieb
- Einfacher Austausch von verschleissbehafteten Werzeugeinsätzen, wobei alle verschleissbehafteten Teile in ein austauschbares Element zusammengefasst sind. Verschleiss-Punkte sind Lagerbüchse und Schaft des Schleifwerkzeuges, Schleiffläche, flexible Welle, Kupplungsstück.
- Da der Auflagedruck durch den langen Hebel des langen Geräteschaftes begrenzt ist, muss die hohe Abtragsleistung durch hohe Drehzahlen erreicht werden.
- Durch die geforderte hohe Drehzahl entstehen Probleme, die gelöst werden müssen durch vibrationshemmende Massnahmen wie dynamische Balancierung, Dämpfung und reibungshemmende Massnahmen. Dies wird durch die Miniaturisierung erreicht: kleine Welle, kleine Lager, kleine Fräser, kleine Kupplung, kleiner Motor.
- Kombination obiger drei Grundgedanken zu einem Austauschwerkzeugeinsatz
- Verwendung der Austauschwerkzeugeinsätze mit den entsprechenden Bearbeitungsgeräten

### Lösung der Aufgabe

Die Aufgabe der vorliegenden Erfindung wird mit einem Werkzeugeinsatz gelöst, der die in Anspruch 1 angegebenen Merkmale aufweist. Die Aufgabe wird auch von einem Bearbeitungsgerät gelöst, das die Merkmale des Anspruchs 7 aufweist.

### Figuren

Unter Bezugnahme auf die beigefügten Zeichnungen werden nachfolgend Ausführungsbeispiele der Erfindung erläutert. Dabei
zeigen:
- Fig. 1: eine schematische Schnittdarstellung eines Arbeitsschaftes eines Bearbeitungsgeräts mit einem Austauschwerkzeug;
- Fig. 2: eine schematische Schnittdarstellung eines gebogenen Arbeitsschaftes;
- Fig. 3: eine schematische Darstellung eines Bearbeitungsgeräts mit einem starren Arbeitsschaftes; und
- Fig. 4: eine schematische Darstellung eines Bearbeitungsgeräts mit einem flexiblen Arbeitsschaft.

### Ausführungsbeispiele

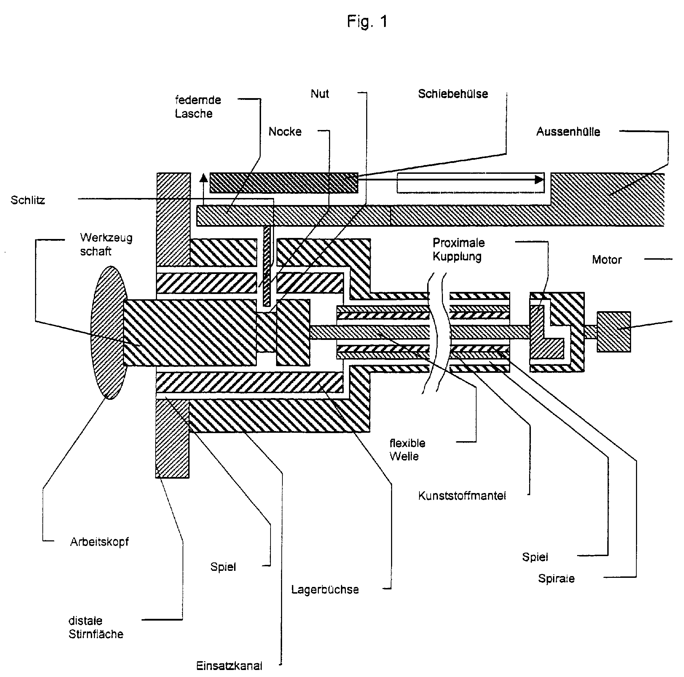

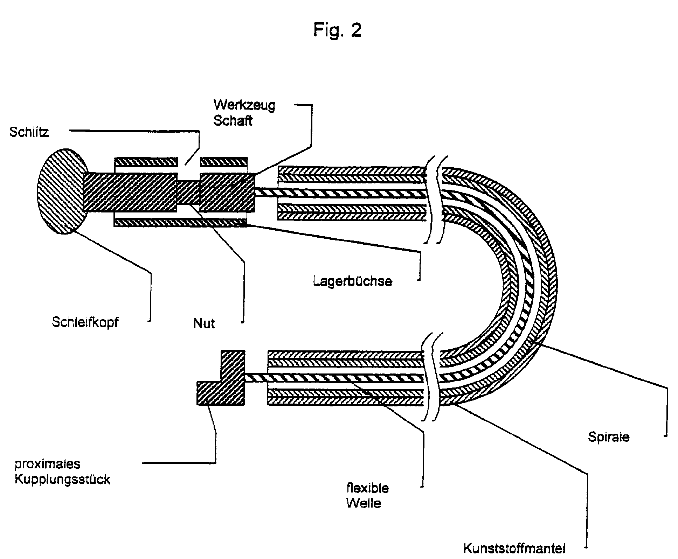

Mit Bezug auf die Fig. 1 und 2 wird nachfolgend ein austauschbarer Werkzeugeinsatz beschrieben.

### Aufbau:

Der kostengünstig herstellbare Austauschwerkzeugeinsatz besteht in einer realisierten Ausführung aus einem Standard-Bearbeitungswerkzeug (Schleifer, Fräser etc.) für den Zahntechnikbedarf, an dessen Schaft eine Stahllitze als flexible Antriebswelle gelötet wurde. Die Litze ist einerseits flexibler als ein massiver Draht, andererseits dämpft die Litze Querschwingungen, die durch Unwuchten entstehen können. Proximal wird das Austauschwerkzeug durch das an die Litze proximal angelötete Kupplungsstück abgeschlossen, das die lösbare Verbindung zum Motor darstellt. Die Litze wird von einer nicht mitrotierenden Spirale umgeben. Zwischen dieser Spirale und der Litze ist möglichst wenig Spiel, so dass sich keine Unwucht aufbauen kann. Eine Unwucht würde neben den störenden Vibrationen auch zu Reibungsverlusten an der Innenseite der Spirale führen. Die Spirale seinerseits ist durch einen Kunststoffschrumpfschlauch ummantelt, der mehrere Funktionen hat. Erstens dient er als Vibrationsdämpfer. Durch eine Dämpfung werden Resonanzen vernichtet, die durch Aufschaukelung der Vibrationen zu Reibungsverlusten führen können. Zweitens schützt er die Spirale vor Verformungen, die durch die Montage oder Demontage entstehen können. Drittens verhindert er einen Schmiermittelverlust.

### Verriegelung:

Der Schaft des Bearbeitungswerkzeuges ist teilweise von einer geschlitzten Lagerbüchse umgeben, die durch den Schlitz, neben der Lagerfunktion, auch Teil eines Verriegelungsmechanismus bildet.

Am Distalende des Bearbeitungsgerätes ist ein Verankerungsmechanismus vorgesehen, welcher die Lagerbüchse und somit den gesamten Austauschwerkzeugeinsatz verankert. Dazu ist beispielsweise am Distalende des Bearbeitungsgerätes eine Schiebehülse angebracht, die in Längsrichtung nach distal und proximal verschoben werden kann. In der einen Position verriegelt sie durch Einbiegen einer nach aussen federnden Lasche, die inwärts mit einer Nocke versehen ist, den Werkzeugschaft an dessen Nut. Auf diese Weise wird eine Verriegelung erreicht mit einem Minimalbedarf an zusätzlichem Schaftdurchmesser. Dabei werden gleichzeitig das rotierende Werkzeug und die Lagerbuchse verriegelt, da letztere einen Schlitz aufweist durch den die Nocke durchdringt und in der umlaufenden Nut des Werkzeuges zur Verriegelung eingreift.

Durch die Verriegelung am Distalende wird bei der flexiblen Welle eine gute Werkzeugführung ermöglicht. Denn wäre das Austauschwerzeug proximal an der Motorkupplung verriegelt, würde durch die Längsfederwirkung der flexiblen Welle das Arbeitswerkzeug bei axialer Anpresskraft nach proximal ausweichen. Dies würde zu einer unpräzise Werkzeughandhabe führen. Die Austauschwerkzeuge des medizinischen Bearbeitungsgerätes der Anmelderin werden proximal verriegelt, was wegen der starren Übertragungswelle aber kaum eine Rolle spielt.

### Vibrationsminimierung:

Die Lagerbüchse hat im Distalende des Bearbeitungsgerätes etwas Spiel, was bewirkt, dass sich Unwuchten des Bearbeitungswerkzeuges nicht stark auswirken, sondern durch eine minime Mitbewegung der Lagerbüchse kompensiert werden. Dies Selbstbalancierung verlängert die Lagerstandzeit, da die reibungsverursachenden Vibrationen minimiert werden.

Ein ähnlicher Effekt tritt beim Übertragungselement auf. Die Spirale wird durch Unwuchten der Litze leicht mitbewegt. Die Bewegung wird durch das Spiel zwischen dem Einschubkanal (Einsatzkanal) und dem Kunststoffschlauch um die Spirale ermöglicht. Durch die leichte Bewegung, die durch den Kunststoffmantel gedämpft wird, tritt eine Selbstbalancierung ein, welche die Vibrationen minimiert.

### Reibungsminimierung:

Da die Bewegungsübertragung über ein gekrümmtes Übertragungselement geschieht, muss zur Reibungsminimierung der Biegemodul des Übertragungselementes möglichst gering sein.

Die Schwungmasse, die vibrieren kann, muss möglichst gering sein. Die Abmessungen einer Asymmterie am Übertragungselement soll möglichst klein sein. Die relative Oberflächengeschwindigkeit Übertragungselement und Schutzhülle muss möglichst gering sein. Die Fläche eines allfälligen Schmierfilms um das Übertragungselement muss möglichst klein sein.

Der Durchmesser des Übertragungselementes geht somit mehrfach in die Reibungsverluste ein. Das Übertragungselement wird wegen den obigen Punkten möglichst mit kleinem Durchmesser gewählt. Da der Anpressdruck des Bearbeitungswerkzeuges meist klein ist, muss auch nur ein kleines Drehmoment übertragen werden. D.h. dass der Durchmesser des Übertragungselementes nur gerade so gross zu sein braucht, dass es das Maximaldrehmoment des Motors übertragen kann.

Die Möglichkeit zur Selbstbalancierung und die Vibrationsdämpfung des Austauschwerkzeuges erlauben problemlos hohe Drehzahlen z.B. über 30'000 UpM. Zudem ist durch die Miniaturisierung des Übertragungselementes die erforderliche Motorleistung minimal, z.B. 2W, was die Verwendung eines kleinen Motors und somit ein graziles Gerätedesign ermöglicht.

Weiter Ausführungsformen der Erfindung benutzen bspw. als Energiequelle Druckluft statt Strom für den Motor, oder setzen als Vibrationsdämpfung der Welle ein Flüssigkeitsbad ein, in welchem die Welle rotiert.

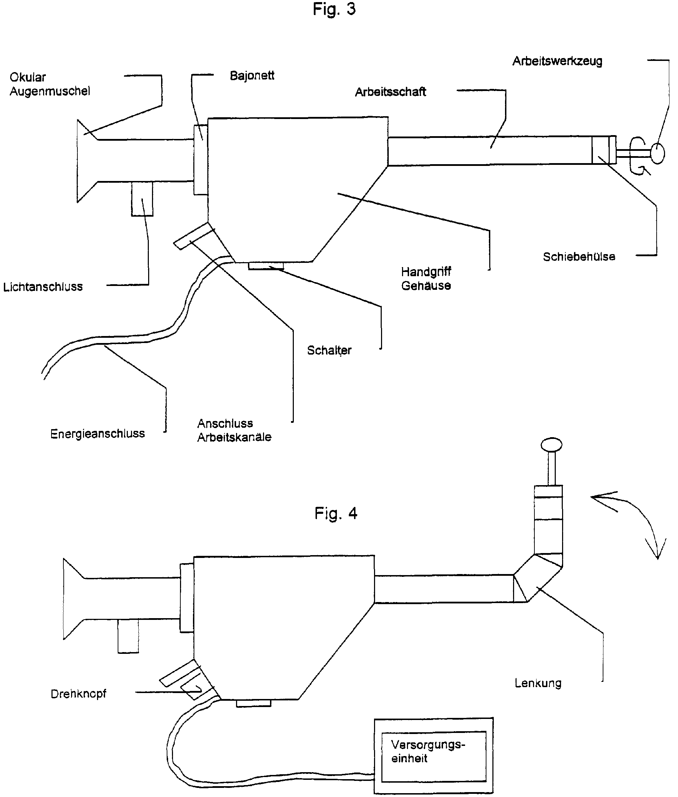

Mit Bezug auf Fig. 3 wird ein endoskopisches Bearbeitungsgerät mit starrem Distalende nachfolgend beschrieben.

Das Bearbeitungsgerät besteht aus einem standardmässigen starren Boroskop mit Blickrichtung 0° bis ca. 30°, welches in einen Arbeitsüberschaft eingeschoben und verriegelt werden kann. Der Arbeitsüberschaft enthält den Aufnahmekanal (Einsatzkanal) für den Austauschwerkzeugeinsatz.

Die Verschleissteile wie Schleifkopf (anwendungsspezifisch), Lagerbüchsen und flexible Welle sind zu einem vom Anwender einfach austauschbaren Zubehörteil, dem Austauschwerkzeugeinsatz, zusammengefasst.

Es sind weitere, universell einsetzbare Arbeitskanäle in den Arbeitsüberschaft integriert. Diese können für Sonden (Fasszangen, angetriebene und ungetriebene Werkzeuge, Sensoren, Zusatzlichtfasern, UV-Analyselicht, UV-Aushärtungslicht, Laser-Bearbeitungsfasern, Faseroptik zur Beobachtung oder Spektroskopie, etc. ) und/oder Medien (Klebstoff, Beschichtungen, Farbe, Chemikalien, Kühlmittel, Schmiermittel, Pulver, Druckluft, Gase, Wasser, etc. ) verwendet werden. Auch die Absaugung von Material aus der Bearbeitungsgegend ist möglich (Schleifstaub, Verschmutzungen, Probenentnahme zur Analyse).

Die Arbeitskanäle sind vorzugsweise mit einem genormten Kupplungsanschluss, wie z.B. Luer-Anschluss versehen. Dort lassen sich neben Zuleitungen auch Sonden etc. verriegeln. Der Ausblick des Boroskopes kommt etwa an die Stirn am Distalende des Arbeitsüberschaftes zu liegen. Proximal am Arbeitsüberschaft ist ein Handgriffgehäuse vorgesehen, welches den Antriebsmotor, Schalter, Elektronik im Innern enthält. Das Gehäuse dient ferner als Basis für die Befestigung und Durchführung der Arbeitskanäle und des Anschlusskabels nach proximal. Das geringe Gewicht und die Form des Handgriffes ermöglichen ein ergonomisches Halten. Die Länge des Griffes wird aber zu Gunsten einer grösseren Arbeitslänge möglichst kurz gehalten.

Das Anschlusskabel wird mit einer multifunktionellen Versorgungseinheit verbunden, welche neben der Versorgungsenergie (primär Elektrizität, aber z.B. auch Druckluft möglich) für den Motor auch z.B. das Beleuchtungslicht für das Endoskop liefern kann.

Zum Betrieb wird Beleuchtungslicht von einer Lichtquelle durch den Lichtanschluss ins Endoskop geleitet. Das am Distalende austretende Licht beleuchtet den Hohlraum und den Schleifkopf. Das Gerät arbeitet mit hoher Tourenzahl, ähnlich einem Zahnarzt-Bohrer, was bei kleinen Auflagekräften grosse Abtragsleistungen und exakte Führbarkeit ermöglicht.

Das Gerät kann entweder mit dem Schalter im Handgriff oder mit einem Fussschalter aktiviert werden. Die Drehzahl kann vorzugsweise eingestellt werden, um sie dem Werkzeug und der Situation anzupassen.

Das Bearbeitungsgerät kann unter Sichtkontrolle an die Bearbeitungsstelle geführt werden, wo sofort mit der Bearbeitung begonnen werden kann.

Durch den modularen Aufbau lässt sich jederzeit das teure Boroskop vom Arbeitsüberschaft leicht trennen. Dies wird durch die einfach lösbare Verbindung mittels des universellen Bajonett-Verschlusses unseres Hauses ermöglicht. Das getrennt einsetzbare Boroskop ermöglicht eine genauere Inspektion der zu bearbeitenden Stelle, da die Optik näher an die Oberfläche zur vergrösserten Detaildarstellung heran geführt werden kann, weil kein Arbeitskopf eines Austauschwerkzeugeinsatzes das Gerät auf Distanz hält.

In Fig. 4 ist das gleiche Bearbeitungsgerät gezeigt wie mit Bezug auf Fig. 3 beschrieben, wobei aber das Distalende mit dem Bearbeitungskopf des Austauscheinsatzwerkzeuges abgelenkt werden kann. Der mechanischen Stabilität zuliebe geht die Ablenkung vorzugsweise in eine Richtung bis zu einem vordefinierten Anschlag, bei dessen Erreichen sich die Lenkung konstruktionsbewusst abrupt versteift. Diese Versteifung verbessert die präzise Werkzeugführung. Die Ablenkung in nur einer Richtung ermöglicht trotzdem eine Bearbeitung eines grossen Bereiches in einem Hohlraum, da das Gerät auch einfach um seine Längsachse mit dem Handgriff rotiert werden kann.

Das Boroskop besitzt in diesem Anwendungsbeispiel im Gegensatz zu der Ausführungsform gemäß Fig. 3 eine Blickrichtung von ca. 70°, wobei der Ausblick vor der Lenkung durch eine Öffnung im Mantel des Arbeitsüberschaftes vorgesehen ist. Die Ablenkung des Distalendes beträgt in diesem Anwendungsbeispiel ca. 90°. Die Abmessungen, die Blickrichtung und das Gesichtsfeld (z.B. 30°) sind so koordiniert, dass der Arbeitskopf an geeigneter Stelle im Bild des Boroskopes zu liegen kommt, z.B. in der Bildmitte oder im unteren Bilddrittel, und so, dass eine angemessene Detailerkennung der zu bearbeitenden Oberfläche ermöglicht wird. Die Winkel für Ablenkung und Gesichtsfeld etc. sind anwendungsbedingt.

Die Ablenkung des Distalendes wird vorzugsweise mit einem relativ kleinen gerändelten Drehknopf am Proximalende des Handgriffgehäuses über einen vorzugsweise bidirektional wirkenden Bowdenzug bewirkt. Im Innern des Drehknopfes befindet sich ein selbsthemmendes Gewinde, das die Rotation des Drehknopfes in eine Translation des Bowdenzuges übersetzt. Anschläge im Drehknopfmechanismus verhindern dabei ein Überdehnen des Bowdenzuges in beide Richtungen.

Das bewegliche Element des bidirektional wirkenden Bowdenzuges ist aus einer Litze und einer sie umgebenden, dicht anliegenden Spirale aufgebaut, die an mindestens den beiden Enden fest miteinander verbunden sind. Der Zug wird dabei durch das Seil, der Schub durch die Spirale übertragen. Ein starres Rohr bildet die Aussenhülle des Bowdenzuges zur Führung und Aufnahme der Gegenkraft.

Durch die Bidirektionalität des Zuges wird Querschnitt eingespart, da zum Strecken und Beugen des Distalendes nur ein Zug benötigt wird, der auf Zug und Schub belastet wird; der bei ablenkbaren Endoskopen übliche Gegenzug entfällt. Durch das Fehlen des Elementes des Gegenzuges erhält man mehr Freiheit für die Wahl und Verteilung der anderen Elemente im Innern des Schaftes. Ferner kann der Drehpunkt der Ablenkglieder asymmetrisch von der Mitte an den dem Zug gegenüberliegenden Ort verlegt werden, was für die Ablenkung eine grössere Hebelwirkung und somit eine grössere Ablenkkraft bewirkt. Mindestens ein Arbeitskanal ist vorsehbar, wie mit Bezug auf Fig. 3 beschrieben.

Das Arbeitsgerät wird in geradem Zustand bis zur ungefähren Arbeitsposition eingeführt. Hier kann eine eingeschobene Faserbildleitersonde eine Einführung unter Sichtkontrolle ermöglichen, da das Boroskop nicht für einen Geradeausblick vorgesehen ist. Nach erreichen der ungefähren Arbeitsposition wird das Distalende mit dem Drehknopf in Arbeitsstellung gebracht, wobei am Ende des Anschlages die vorteilhafte Versteifung eintritt. Eine Variante der Lenkung sieht auch einen Mechanismus vor, der durch ein weiteres Betätigungselement eine Versteifung des flexiblen Teils in beliebiger Ablenkposition ermöglicht, ohne dass die Ablenkung bis zum Anschlag ausgelenkt werden muss.

Wenn die richtige Ablenkposition eingestellt ist, kann der Motor wie in Fig. 3 aktiviert werden und die endoskopische Bearbeitung der Oberfläche unter gleichzeitiger Beobachtung ausgeführt werden.

Das Bearbeitungsgerät wird vorteilhafterweise wieder mit geradegerichteter Ablenkung aus dem Bearbeitungsraum entfernt.

Analog zu der Ausführung gemäß Fig. 3 kann auch hier das eingesetzte Boroskop aus dem Arbeitsüberschaft entnommen werden zur vergrösserten Detailbetrachtung der Oberfläche durch Benutzung des unmontierten Boroskopes.

Eine Modifikation der in Fig. 4 gezeigten Ausführungsform besteht darin, kein starres Boroskop einzusetzen, sondern stattdessen eine Faseroptik zu verwenden, die an der Stirnseite des ablenkbaren Distalendes ausblickt. Der Ablenkmechanismus und der zusätzliche Arbeitskanal ist analog zu der Ausführungsform gemäß Fig. 4 ausgebildet.

Durch die drei Variantenbeispiele kann eine große Vielfalt an Oberflächengeometrien bearbeitet werden.

## Patentansprüche

1. Austauschbarer Werkzeugeinsatz für ein endoskopisches Bearbeitungsgerät, mit einer Welle, die an ihrem distalen Ende mit einer Werkzeugeinheit und an ihrem proximalen Ende mit einem Kupplungselement verbunden ist, **dadurch gekennzeichnet, daß** die Welle flexibel ausgebildet ist, und die Werkzeugeinheit ein Verriegelungselement aufweist, das mit einem Verriegelungsteil des Bearbeitungsgeräts zusammenwirkt, um den Werkzeugeinsatz lösbar zu halten.

2. Werkzeugeinsatz nach Anspruch 1, **dadurch gekennzeichnet, daß** die Werkzeugeinheit einen Arbeitskopf und einen Werkzeugschaft umfaßt, und daß das Verriegelungselement als umlaufende Nut in dem Werkzeugschaft ausgebildet ist.

3. Werkzeugeinsatz nach Anspruch 2, **dadurch gekennzeichnet, daß** eine Lagerbuchse vorgesehen ist, die einen Längsabschnitt des Werkzeugschafts aufnimmt und einen Schlitz aufweist, der so ausgebildet ist, daß das Verriegelungselement den Schlitz durchgreifen kann.

4. Werkzeugeinsatz nach einem der vorhergehenden Ansprüche, **dadurch gekennzeichnet, daß** die Welle als Litze ausgebildet ist.

5. Werkzeugeinsatz nach einem der vorhergehenden Ansprüche, **dadurch gekennzeichnet, daß** eine Spirale vorgesehen ist, die einen Längsabschnitt der Welle drehbar aufnimmt.

6. Werkzeugeinsatz nach Anspruch 5, **dadurch gekennzeichnet, daß** ein Mantel, vorzugsweise einen Kunststoffmantel vorgesehen ist, der die Spirale umgibt.

7. Endoskopisches Bearbeitungsgerät mit einem Werkzeugeinsatz nach einem der Ansprüche 1 bis 6, **gekennzeichnet durch** eine Verriegelungseinheit am distalen Ende des Bearbeitungsgeräts, wobei die Verriegelungseinheit ein Verriegelungsteil umfaßt, das mit dem Verriegelungselement des Werkzeugeinsatzes zusammenwirkt.

8. Bearbeitungsgerät nach Anspruch 7, **dadurch gekennzeichnet, daß** sich das Verriegelungsteil senkrecht zur Längsachse des Endoskops erstreckt und an seinem der Längsachse abgewandten Ende an einem parallel zur Längsachse verlaufenden Halteteil der Verriegelungseinheit angebracht ist.

9. Bearbeitungsgerät nach Anspruch 8, **dadurch gekennzeichnet, daß** das Halteteil als im wesentlichen senkrecht zur Längsachse federnde Lasche ausgebildet ist, so daß das Verriegelungsteil senkrecht zur Längsachse bewegbar ist.

10. Bearbeitungsgerät nach Anspruch 9, **dadurch gekennzeichnet, daß** die Verriegelungseinheit eine in axialer Richtung verlagerbare Hülse aufweist, die über die Lasche schiebbar ausgebildet ist und das Verriegelungsteil senkrecht zur Längsachse bewegt, so daß das Verriegelungsteil in Eingriff mit dem Verriegelungselement des Werkzeugeinsatzes bringbar ist.

11. Bearbeitungsgerät nach einem der Ansprüche 7 bis 10, **dadurch gekennzeichnet, daß** ein Einsatzkanal vorgesehen ist, der den Werkzeugeinsatz mit Spiel aufnimmt, derart daß der Werkzeugeinsatz in axialer Richtung in den Einsatzkanal ein- und ausführbar ist.

12. Bearbeitungsgerät nach einem der Ansprüche 7 bis 11, **gekennzeichnet durch** eine Versorgungseinheit für Licht und Energie.
